# EUROPÄISCHE PATENTSCHRIFT

(11) **EP 3 125 988 B1**
(45) Veröffentlichungstag und Bekanntmachung des Hinweises auf die Patenterteilung: **14.06.2017**
(21) Anmeldenummer: 15712032.0
(22) Anmeldetag: 23.02.2015
(51) Int. Cl.: A61N 1/04, A61B 5/0408, A61N 1/08, A61B 50/30, A61B 50/31, A61N 1/372

(54) **ELEKTRODENSET, INSBESONDERE FÜR EINEN DEFIBRILLATOR**
ELECTRODE SET, IN PARTICULAR FOR A DEFIBRILLATOR
JEU D'ÉLECTRODES, EN PARTICULIER POUR UN DÉFIBRILLATEUR

(30) Priorität: 27.03.2014 AT 2192014
(43) Veröffentlichungstag der Anmeldung: 08.02.2017
(73) Patentinhaber: Leonh. Lang, 6020 Innsbruck (AT)
(72) Erfinder: LANG, Burrhus, 6020 Innsbruck (AT); FÖGER, Simon, 6410 Telfs (AT)
(74) Vertreter: Torggler & Hofinger Patentanwälte
(86) Internationale Anmeldenummer: PCT/AT2015/000031
(87) Internationale Veröffentlichungsnummer: WO 2015/143460

(56) Entgegenhaltungen:
- DE-A1- 10 255 919
- US-A1- 2006 142 810
- US-A1- 2007 203 558
- US-A1- 2008 210 592
- US-A1- 2010 094 388

## Beschreibung

Die Erfindung betrifft ein Elektrodenset, insbesondere für einen Defibrillator, beinhaltend zumindest zwei Elektroden, jeweils bestehend aus
- einer Trägerschicht,
- einer leitfähigen Kontaktschicht,
- einer leitfähigen Gelschicht,
- einer nichtleitenden Elektrodenabdeckung, wobei im Lagerzustand des Elektrodensets die Elektrodenabdeckungen, auf der von der Gelschicht der zumindest zwei Elektroden abgewandten Seite, zumindest abschnittsweise flächig aneinander liegen.

Weiters soll ein Verfahren zur Herstellung des erfindungsgemäßen Elektrodensets und ein Verfahren zur Prüfung eines Leitwertes einer Gelschicht in einem Elektrodenset angegeben werden.

Derartige Elektrodensets zählen bereits zum Stand der Technik und werden beispielsweise in der US Patentschrift 5,579,919 gezeigt. Weitere gattungsgemäße Elektrodensets zeigen beispielsweise die US 2007/0203558 A1 und die US 2010/0094388 A1

Der Stand der Technik umfasst diverse Elektrodensets, deren Elektroden sich zum Schutz in einer gasdichten Verpackung befinden. Die an den Elektroden angebrachten Stromleiter ragen dabei aus der gasdichten Verpackung heraus, um an eine elektrische Einrichtung, wie beispielsweise einem Defibrillator, angeschlossen werden zu können.

In der Vergangenheit wurden, besonders für Laiendefibrillatoren, gasdicht verpackte Elektroden verwendet, welche über Kontaktflächen oder zusätzlich angebrachte Prüfkontakte eine sogenannte "Inline-Messung" durchführen konnten. Diese "Inline-Messung" dient dazu, den Leitwert der auf der Elektrode aufgebrachten Gelschicht zu kontrollieren. Manche Elektrodensets weisen keine zusätzlich angebrachten Kontakte für die Inline Messung auf - bei diesen wird der Leitwert direkt über durch die Gelschicht ausgeformte Kontaktflächen geprüft. Diese Kontaktfläche wird durch eine oder mehrere Ausnehmungen in der zwischen den Elektroden angeordneten, isolierenden Elektrodenabdeckung hergestellt.

Wird eine Elektrode verwendet, muss diese aus der Verpackung entnommen und mit der Gelschicht am Körper einer betroffenen Person befestigt werden. Diese klebende Gelschicht ist zeitgleich auch ein Leiter für den elektrischen Strom, der im Ernstfall durch den Defibrillator abgegeben wird. Bei längerer Lagerung kann es sein, dass diese Gelschicht beispielsweise durch Austrocknung ihre Leitfähigkeit verliert. Dies würde bei der Anwendung der Elektrode zu schwerwiegenden Problemen führen. Aus diesem Grund wird durch die Inline-Messung periodisch über den Defibrillator geprüft, ob der Leitwert der Gelschicht in Ordnung ist.

Die Inline Messung erfolgt über einen Kontrollstrom, der vom Defibrillator aus über Stromleiter in die Verpackung der Elektroden gesendet wird, dort durch die Elektrode durchläuft, über eine Kontaktfläche mit der anderen Elektrode fließen kann und über einen zweiten Stromleiter zurück zum Defibrillator gelangt. Ist der Kontakt zwischen den Elektroden nicht ausreichend gut, gibt der Defibrillator eine Warnung ab. Ausschlaggebend für die Funktion dieser Inline-Messung ist natürlich, dass der Kontakt zwischen den zwei Elektroden an der Oberfläche der Gelschicht erfolgt, da diese anhand ihrer Leitfähigkeit geprüft werden sollte.

In einem konkreten Ausführungsbeispiel wird auf eine Elektrodenabdeckung beidseitig je eine Elektrode aufgebracht. Dies gestaltet das Montageverfahren als aufwändig, da bei der Produktion oder der Montage der Elektrodensets die Elektrodenabdeckung während des laufenden Prozesses gedreht oder deren Unterseite auf eine andere Art und Weise zugänglich gemacht werden muss. Daraus resultieren zusätzliche Arbeitsschritte und höhere Produktionskosten.

Aufgabe der Erfindung ist es, die vorbeschriebenen Nachteile zu vermeiden und ein gegenüber dem Stand der Technik verbessertes Elektrodenset bzw. Verfahren zur Herstellung eines Elektrodensets anzugeben.

Diese Aufgabe wird durch die Merkmale der Ansprüche 1, 14 und 15 gelöst.

Als besonders vorteilhaft hat es sich dabei herausgestellt, wenn für jede Elektrode je eine gesonderte Elektrodenabdeckung vorgesehen ist. Die Elektrodenabdeckungen werden vor dem Verpacken zusammengelegt, die Elektroden befinden sich dann an der Außenseite der zusammengelegten Elektrodenabdeckungen. Auch die Produktionskosten der Elektrodensets können gesenkt werden: da pro Elektrode eine Elektrodenabdeckung vorgesehen ist, muss die Elektrodenabdeckung im Produktionsprozess nicht gewendet werden, um diese beidseitig mit Elektroden zu bestücken. Die auf den Elektrodenabdeckungen aufgeklebten Elektroden werden nach der Produktion paarweise miteinander verpackt.

Gemäß einem weiteren Ausführungsbeispiel kann vorgesehen sein, dass die Elektrodenabdeckungen eine gemeinsame - vorzugsweise einstückige - Elektrodenabdeckung bilden. Durch die einstückige Variante ist es möglich, beide Elektroden auf einer Elektrodenabdeckung zu befestigen und diese im Anschluss zu verpacken. Werden beide Elektroden auf derselben Seite aufgeklebt - wie es die Erfindung vorsieht - kann bei der Produktion der Elektrodensets auf ein Wenden der Elektrodenabdeckungen verzichtet werden. Die Bestückung der Elektrodenabdeckungen mit den Elektroden in der Produktion, vorzugsweise auf einem Fertigungsband, erfolgt einfach und schnell von nur einer Seite - beispielsweise von Oben.

Wenn die zumindest abschnittsweise im Wesentlichen ebenen Elektrodenabdeckungen miteinander über eine Faltlinie ständig in Verbindung stehen, können die Elektrodenabdeckungen zueinander zusammengefaltet werden. Dies führt dazu, dass die zusammengefalteten Elektrodenabdeckungen eine kleinere Verpackung benötigen und auch platzsparend am Defibrillator angebracht werden können.

Gemäß einem weiteren Ausführungsbeispiel kann vorgesehen sein, dass die Faltlinie eine zumindest abschnittsweise gefertigte Perforierung der Elektrodenabdeckung ist. Somit ist beim Falten der Elektrodenabdeckung gewährleistet, dass die Elektroden nach dem Zusammenklappen sauber übereinander liegen und die Schnittkanten sowie Ausnehmungen oder Positionierungshilfen der Elektrodenabdeckung miteinander fluchten.

Wenn die in Verbindung stehenden Elektrodenabdeckungen zusammenfaltbar sind und sich im Lagerzustand zwischen den Elektroden befinden ist gewährleistet, dass sich die Elektroden an der Außenseite des ausgepackten Elektrodensets befinden. Die Elektroden können sich somit nicht ungewollt in der Verpackung berühren und eventuell Fehler bei der Inline-Messung verursachen, da Kontakte aufeinander treffen.

Gemäß eines weiteren Ausführungsbeispieles kann vorgesehen sein, dass die Gesamtoberfläche der Elektrodenabdeckungen zumindest das Zweifache der Fläche der Gelschichten der Elektroden beträgt und jene Flächen der Elektrodenabdeckungen - gegenüberliegend der Flächen, auf welchen die Elektroden befestigt sind - frei von Elektroden sind. Daraus ergibt sich wie bereits erwähnt der Effekt, dass sich die Elektroden untereinander nicht ungewollt berühren können, da genügend freie Fläche entsteht, die sich zwischen den Elektroden befindet. Dies gilt sowohl für die Einstückige Variante, die zusammenfaltbar ist als auch für die "zweistückige" Variante mit den gesonderten Elektrodenabdeckungen. Bei beiden Varianten werden die Elektrodenabdeckungen aneinanderliegend verpackt und die Elektroden liegen an den Außenseiten der Elektrodensets. Ein Kontakt der Gelschichten (und der Elektroden zueinander) ist somit ausgeschlossen, außer dieser ist - beispielsweise durch Ausnehmungen in den Elektrodenabdeckungen - erwünscht.

Im Bereich der Gelschicht einer jeden Elektrode gibt es eine Ausnehmung in der Elektrodenabdeckung. Durch diese Ausnehmung können die Gelschichten der Elektroden bei einem Zusammenlegen der Elektrodenabdeckungen einen Kontakt zueinander aufbauen indem sie sich berühren. Somit kann eine Inline - Messung ermöglicht werden, ohne Zusatzkontakte an den Elektroden anbringen zu müssen oder - unter Verwendung von nur einer Elektrodenabdeckung samt Ausnehmung mit beidseitiger Bestückung durch eine Elektrode - bei der beidseitigen Bestückung ein aufwendiges Wenden der Elektrodenabdeckung im Produktionsprozess durchführen zu müssen.

Durch Positionierungshilfen, wie vorzugsweise Führungslöcher oder Ausformungen an der Außenkante der Elektrodenabdeckungen, die sich außerhalb des Aufnahmebereiches der Elektroden befinden, wird im Produktionsprozess beim Zusammenlegen der Elektrodenabdeckungen eine exakte Positionierung der Ausnehmungen zueinander gewährleistet.

Als besonders vorteilhaft hat es sich dabei herausgestellt, wenn die einteilige Elektrodenabdeckung zumindest eine große Ausnehmung in einem Aufnahmebereich und zumindest eine kleine Ausnehmung in einem weiteren Aufnahmebereich aufweist. Somit wird nach dem Zusammenklappen oder Zusammenlegen der Elektrodenabdeckung gewährleistet, dass die kleinere Ausnehmung immer im Bereich der größeren Ausnehmung Platz findet und eine saubere Kontaktfläche zwischen den zwei Gelschichten der beiden Elektroden gewährleistet ist. Ein Überschneiden der Ausnehmungen ist aufgrund der unterschiedlichen Größe somit nicht möglich, wenn die Elektrodenabdeckung an der Faltlinie geknickt oder über die Positionierungshilfen geführt zusammengefaltet wird. Aus den unterschiedlich großen Ausnehmungen ergibt sich der Vorteil, dass das Zusammenfalten oder Zusammenlegen der Elektroden nicht so präzise ausgestaltet sein muss. Dies spiegelt sich in einer effizienteren Produktionsgeschwindigkeit und einer geringeren Ausschussrate wieder.

Wenn im Lagerzustand des Elektrodensets die zumindest eine kleine Ausnehmung mit der zumindest einen großen Ausnehmung fluchtet und dadurch die Gelschichten der Elektroden in Kontakt zueinander stehen, wird die Kontaktfläche durch die kleine Ausnehmung bestimmt. Durch die Verbindung der Gelflächen der zwei Elektroden über die kleine Ausnehmung in der Elektrodenabdeckung entsteht an der kleinen Ausnehmung der Elektrodenabdeckung ein elektrischer Widerstand sobald Strom über die Elektroden fließt. Über die Dimensionierung der Größe der kleinen Ausnehmung kann somit ein Fixwert für die Prüfung der Leitfähigkeit der Gelschichten an den Elektroden eingestellt werden. Dieser Fixwert, der sich aus der Größe der kleinen Ausnehmung ergibt, ist in jeder verpackten Einheit des Elektrodensets gleich groß. Zusätzlich stellt im Verpackungszustand die Flächendifferenz zwischen der kleinen Ausnehmung und der großen Ausnehmung in ihrer Dimensionierung die Verbindungsfläche der einen Kombination aus Elektrode und Elektrodenabdeckung zur anderen Kombination aus Elektrode und Elektrodenabdeckung dar. Vorteilhaft ist, dass die Verwendung eines zusätzlichen Klebefilms oder Verbindungsmittels entfällt, da die Klebeeigenschaft der Gelschichten verwendet werden kann. Somit ist - ohne Verwendung zusätzlicher Mittel wie Klebemittel oder ähnlichem - gewährleistet, dass die zwei Kombinationen aus Elektrode und Elektrodenabdeckung sich in der Verpackung nicht voneinander trennen können.

Gemäß einem weiteren Ausführungsbeispiel kann vorgesehen sein, dass sich im Lagerzustand die zumindest eine kleine Ausnehmung vollständig im Bereich der zumindest einen großen Ausnehmung befindet, um keine Überschneidungen der zwei Ausnehmungen zueinander zu ermöglichen. Dies erfolgt nicht nur über die unterschiedliche Größe der Ausnehmungen, sondern beispielsweise auch über die Faltlinie/Perforierung oder eine Positionierungshilfe. Durch das Falten an der Faltlinie/Perforierung wird die eine Ausnehmung über die weitere Ausnehmung gelegt. Beim Zusammenlegen einer "zweistückigen" Ausführung mit gesonderten Elektrodenabdeckungen erfolgt die genaue Positionierung der Ausnehmungen zueinander zum Beispiel durch die Verwendung der Positionierungshilfen. Die unterschiedliche Größe der Ausnehmungen gibt zusätzliche Sicherheit, dass die Ausnehmungen auch tatsächlich miteinander fluchten. Dies ist zu erwarten, wenn die Fläche der zumindest einen kleinen Ausnehmung 30 - 70 % der Fläche der zumindest großen Ausnehmung beträgt.

Gemäß eines weiteren Ausführungsbeispiels kann vorgesehen sein, dass die Ausnehmungen vorzugsweise durch Kreise ausgeformt sind, da diese einfach in der Produktion herstellbar sind und beim Zusammenfalten über die Faltlinie leichter zueinander fluchten, als beispielsweise Drei- oder Vielecke.

Als besonders vorteilhaft hat es sich herausgestellt, wenn zumindest eine Positionierungshilfe durch die Elektrodenabdeckungen ausgeformt ist. Durch diese Positionierungshilfe, vorzugsweise durch Löcher oder der Form der Elektrodenabdeckungen ausgeformt, können bei der Montage der Elektrodensets Fehler vermieden werden. Ein falsches Zusammenlegen der Elektrodensets wird somit verhindert und eine exakte Positionierung der in den Elektrodenabdeckungen befindlichen Ausnehmungen zueinander gewährleistet.

Gemäß einem weiteren Ausführungsbeispiel kann vorgesehen sein, dass das Fertigen der Ausnehmungen in das Material der Elektrodenabdeckung vor dem Aufkleben der Klebeelektroden auf das Material der Elektrodenabdeckung erfolgt. Dies vereinfacht den Stanzprozess und gewährleistet, dass die Anordnungen der Ausnehmungen zueinander immer präzise gestanzt ist, was bei der Inline-Messung notwendig ist.

Gemäß einem weiteren Ausführungsbeispiel kann vorgesehen sein, dass der Laser und/oder die Lochstanze, die Elektrodenstanze und die Kartenstanze Teile einer automatisierten Fertigungsanlage zur Herstellung des Elektrodensets sind, was eine wirtschaftliche Produktion der Elektrodensets ermöglicht.

Als besonders vorteilhaft hat es sich dabei herausgestellt, wenn ein automatischer Vorschub das Material für die Elektrodenabdeckung und die Klebeelektroden durch die Fertigungsanlage führt. Dies trägt auch einen wesentlichen Teil zur effizienteren und schnelleren Produktion der Elektrodensets bei.

Gemäß einem weiteren Aspekt der Erfindung kann vorgesehen sein, dass ein Kontrollstrom ausgehend von einer elektrischen Vorrichtung, vorzugsweise einem Defibrillator, durch das im Lagerzustand befindliche, verpackte Elektrodenset geleitet wird, wobei sich die zumindest eine kleine Ausnehmung vollständig im Bereich der zumindest einen großen Ausnehmung befindet und somit eine durch die Fläche der kleinen Ausnehmung definierte Kontaktfläche der Elektroden zueinander bildet, durch welche der Kontrollstrom die Elektrodenabdeckung passieren kann und der Leitwert ermittelbar wird.

Weitere Einzelheiten und Vorteile der vorliegenden Erfindung werden anhand der Figurenbeschreibungen unter Bezugnahme auf die in den Zeichnungen dargestellten Ausführungsbeispiele im Folgenden näher erläutert. Darin zeigen:
- Fig. 1: skizzierte Darstellung der Bestandteile einer Elektrode in eine Explosionszeichnung,
- Fig. 2: Elektrodenabdeckung in der Draufsicht,
- Fig. 3a und 3b: skizzierte Darstellung des Fertigungsablaufs beim Stanzen der Elektroden auf der Elektrodenabdeckung,
- Fig. 4: skizzierte Darstellung eines Elektrodensets ohne Verpackung im aufgeklappten Zustand,
- Fig. 5: skizzierte Schnittdarstellung eines Elektrodensets im verpackten Zustand,
- Fig. 6: skizzierte Darstellung eines Elektrodensets, angeschlossen an einen Defibrillator und
- Fig. 7: skizzierte Darstellung des Stromflusses beim Prüfvorgang (Inline-Messung) eines Elektrodensets.
- Fig. 8: skizzierte Darstellung eines Elektrodensets ohne Verpackung auf einzelnen Elektrodenabdeckungen.

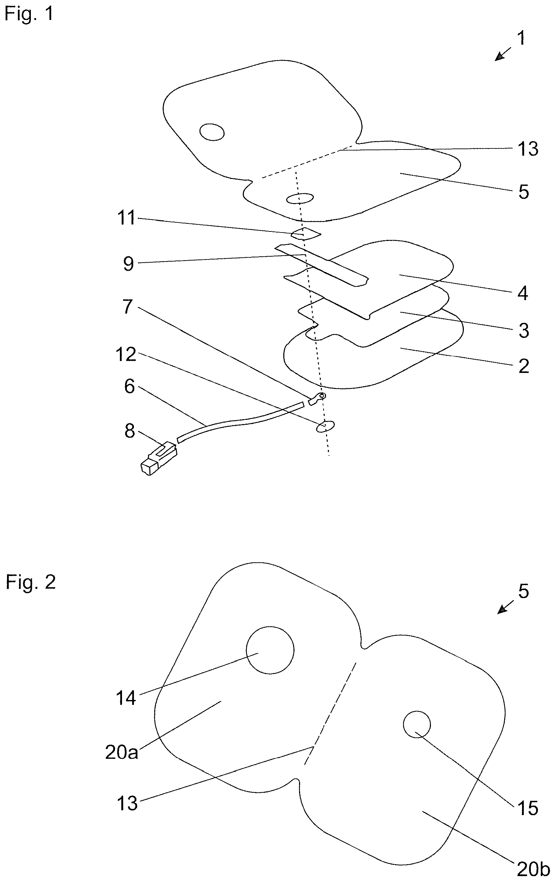

Die Figur 1 zeigt eine von normalerweise paarweise angeordneten Elektroden in einer Explosionszeichnung, bestehend aus einer Trägerschicht 2, vorzugsweise aus einem dünnen Schaumstoff gefertigt, einer Kontaktschicht 3, vorzugsweise aus einer Metallfolie gefertigt, die elektrisch leitend ist und mit dem Kontaktelement 7 verbunden ist. Das Kontaktelement 7 ist leitend an einem Stromleiter 6 befestigt, welcher an seinem Ende einen Stecker 8 aufweist. An der Kontaktschicht 3 befindet sich die Gelschicht 4. Die Gelschicht 4 besteht aus einem klebenden, elektrisch leitfähigen Material und stellt bei der Verwendung eines Defibrillators die Verbindungen zwischen dem Körper und der Elektrode her. Zur Verbindung der einzelnen Schichten zueinander wird ein Klebeelement 9 verwendet. Das Isolierelement 11 und das Abdeckungselement 12 dienen zur Isolierung und Abdeckung beispielsweise einer leitfähigen Niete, die die Verbindung zwischen dem Kontaktelement 7 und der Kontaktschicht 3 herstellt. Die Elektrodenabdeckung 5 wird über die Gelschicht 4 der Elektrode 1 geklebt und schützt die Gelschicht 4 vor Verkleben mit der zweiten Elektrode, welche in der Figur 1 nicht ersichtlich ist.

Weiters weist die Elektrodenabdeckung 5 eine Faltlinie 13 auf, welche ein Knicken, oder in anderen Worten auch Falten, der Elektrodenabdeckung ermöglicht.

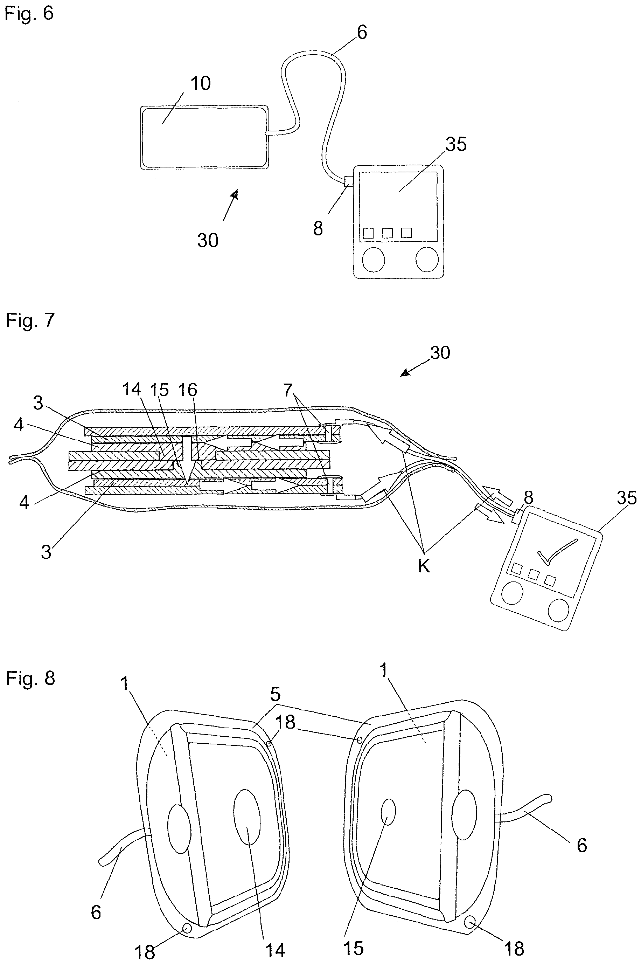

Figur 2 zeigt eine Elektrodenabdeckung 5 mit einer Faltlinie 13, die hier durch eine Perforierung in Form einer unterbrochenen Linie hergestellt wurde. Über die Faltlinie 13 wird die Elektrodenabdeckung 5 in zwei Bereiche aufgeteilt, zum Einen in den Aufnahmebereich 20a und zum Anderen in den Aufnahmebereich 20b. Auf jedem dieser Aufnahmebereiche 20a, 20b wird in weiterer Folge eine Elektrode platziert. Diese sind in der Figur 2 noch nicht ersichtlich. Im Aufnahmebereich 20a und 20b befindet sich je eine Ausnehmung 14, 15. Diese sind so angeordnet, dass nach dem Falten der Elektrodenabdeckung 5 über die Faltlinie 13 die Ausnehmung 15 in der mit der Ausnehmung 14 fluchtet. Wird eine Ausnehmung 14, 15 größer als die andere dimensioniert, so verringert sich die Möglichkeit, dass die Ausnehmungen 14, 15 nach dem Zusammenfalten nicht fluchten. Wird die Elektrodenabdeckung 5 zusammengefaltet kann beispielsweise die kleine Ausnehmung 15 in der großen Ausnehmung 14 Platz finden und von dieser vollständig eingenommen werden. Würde die Elektrodenabdeckung zweiteilig ausgeführt (siehe Fig. 8), so würden zwei unterschiedlich große Ausnehmungen 14, 15 den Montageprozess ebenfalls vereinfachen - es ist eine geringere Präzision beim Zusammenlegen oder Zusammenfalten notwendig. Somit kann das Montageverfahren schneller und günstiger gestaltet werden.

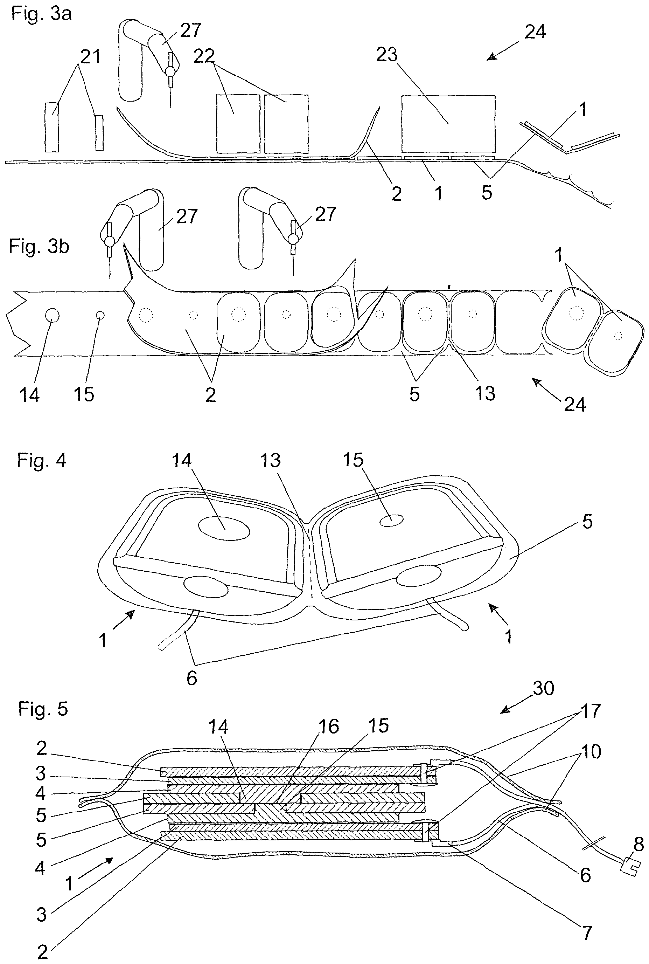

Die Figur 3a zeigt schematisch dargestellt den Fertigungsprozess der Elektrode 1. In einem Fertigungsabschnitt werden aus einem in einer Bahn verlaufenden Material für die Elektrodenabdeckungen mit den Lochstanzen 21 die zwei Ausnehmungen 14, 15 herausgestanzt.

In einem weiteren Arbeitsschritt wird in einer Bahn die vorbereitete Elektrode 1 auf das Material der Elektrodenabdeckung aufgeklebt. In einem nächsten Arbeitsschritt werden mittels einer Elektrodenstanze 22 entweder nacheinander oder zeitgleich eine oder mehrere Elektroden 1 aus dem Material für die Elektroden ausgestanzt, geschnitten oder gelasert. In einem weiteren Arbeitsschritt wird die restliche, nicht mehr notwendige Folie der Trägerschicht 2 der Elektrode 1 abgezogen. Die Elektroden 1 befinden sich noch mit ihrer Gelschicht 4 korrekt über den Ausnehmungen 14, 15 platziert auf dem Material für die Elektrodenabdeckung 5.

In einem weiteren Arbeitsschritt wird nun über die Kartenstanze 23 oder einem Laser 27 ein Elektrodenpaar aus dem Material der Elektrodenabdeckungen ausgeschnitten. Das Restmaterial der Folie für die Elektrodenabdeckungen 5 wird im nächsten Arbeitsschritt abgeführt. Die Perforierung der Faltlinie 13 in der Elektrodenabdeckung 5 kann gelasert oder in einem Arbeitsschritt mit dem Stanzen durch die Kartenstanze 23 erfolgen beziehungsweise auch nachher in einem weiteren Schritt.

Die Figur 3b zeigt dieselben Arbeitsschritte wie die Figur 3a in einer Draufsicht. Die Ausnehmungen 14, 15 sind nach dem Auftragen der Trägerschicht 2 für die Elektroden 1 nur noch strichliert dargestellt, da diese sonst in der schematischen Darstellung nicht mehr sichtbar gewesen wären. Die Perforierung der Faltlinie 13 erfolgt bei dieser Darstellung zeitgleich mit dem Stanzen der Kartenstanze 23 oder kann gleich wie die Umrisse der Elektrodenabdeckungen 5 mittels Laser 27 geschnitten werden.

Figur 4 zeigt ein unverpacktes Elektrodenset 30 auf einer Elektrodenabdeckung 5. Die hier transparent dargestellte Elektrodenabdeckung 5 ist in dieser schematischen Darstellung die oberste Schicht. Die Klebeelektroden sind an der Unterseite der Elektrodenabdeckung 5 aufgeklebt. In diesem aufgefalteten Zustand der Elektrodenabdeckung 5 steht die kleine Ausnehmung 15 nicht in Kontakt mit der großen Ausnehmung 14. Sobald die Elektrodenabdeckung 5 über die Faltlinie 13, hier als strichlierte Perforierung dargestellt, zusammengefaltet wird, stehen die Gelschichten 4 der Elektroden über die kleine Ausnehmung 15 zueinander in Verbindung.

Die Figur 5 zeigt eine schematische Darstellung eines verpackten Elektrodensets 30 in einer Schnittdarstellung. Die Verpackung 10 ummantelt dabei die komplette Einheit bestehend aus Elektroden 1 und Elektrodenabdeckung 5. Der Stromleiter 6 wird dabei gasdicht aus der Verpackung geführt. Über Verbindungselemente 17 steht der Stromleiter 6 mit der Kontaktschicht 3 in Verbindung. Der aus der gasdichten Verpackung 10 herausgeführte Stromleiter 6, weist an einem Ende einen Stecker 8 auf. Mit diesem Stecker kann das Elektrodenset 30 an einen Defibrillator 35 angeschlossen werden. Zwischen den zwei Elektroden 1 befindet sich die Elektrodenabdeckung 5 im zusammengefalteten Zustand, was hier durch zwei Schichten angedeutet wird. In jeder dieser aus der Elektrodenabdeckung bestehenden Schicht befindet sich eine Ausnehmung 14, 15. Über diese Ausnehmung 14, 15 nehmen die Gelschichten 4 der Elektroden 1 Kontakt zueinander auf. Die somit entstehende Kontaktfläche zwischen den Gelschichten 4, in anderen Worten auch Prüfkontakt 16 genannt, stellt die einzige Verbindung zwischen den zwei Gelschichten 4 der verpackten Elektroden 1 dar.

Die Figur 6 zeigt ein Elektrodenset 30 im angeschlossen Zustand über einen Stromleiter 6 und einen Stecker 8 an einen Defibrillator 35. In diesem Zustand kann vom Defibrillator 35 aus ein Prüfstrom an das Elektrodenset 30 entsandt und wieder empfangen werden. Gäbe es ein Problem mit der Leitfähigkeit der Gelschicht 4 der verpackten Elektroden 1, würde der Defibrillator 35 eine Fehlermeldung abgeben.

Die Figur 7 zeigt schematisch den Verlauf des Kontrollstromes K ausgehend vom Defibrillator 35 durch den Stecker 8 in den Stromleiter 6, vorzugsweise ein zweipoliges Kabel, in das Elektrodenset 30. Im Elektrodenset 30 fließt der Kontrollstrom K über das Kontaktelement 7 in die Kontaktschicht 3, die mit der Gelschicht 4 verbunden ist. Der durch die Ausnehmungen 14, 15 gebildete Prüfkontakt 16 erlaubt es dem Kontrollstrom K, als Pfeil dargestellt, von der einen Gelschicht 4 in die andere Gelschicht 4 zu fließen. In weiterer Folge geht der Kontrollstrom K seinen Weg weiter zum Verbindungselement 7 und in das zweipolige Kabel zurück, welches über den Stecker 8 mit dem Defibrillator 35 verbunden ist. Kann der Kontrollstrom K ohne größeren Widerstand durch das Elektrodenset 30 fließen, in anderen Worten: War die Inline-Messung erfolgreich, kann das Elektrodenset 30 weiterhin verwendet werden und muss nicht ausgetauscht werden. Ist die Leitfähigkeit der Gelschicht 4 nicht mehr ausreichend, was in Folge einer mechanischen Beschädigung der Verpackung oder des gesamten Elektrodensets 30 passieren könnte, wird dies als Fehler am Defibrillator 35 registriert. In diesem Fall muss das Elektrodenset 30 ausgetauscht werden.

Die Figur 8 zeigt ein unverpacktes Elektrodenset 30 mit je einer Elektrode 1 auf je einer Elektrodenabdeckung 5. In diesem Ausführungsbeispiel sind die Elektrodenabdeckungen nicht einstückig ausgeführt. Die hier transparent gezeichneten Elektrodenabdeckungen 5 liegen schematisch dargestellt vor den Elektroden 1 und verdecken die Elektroden 1. In diesem nicht zusammengelegten Zustand der Elektrodenabdeckungen 5 steht die kleine Ausnehmung 15 nicht in Kontakt mit der großen Ausnehmung 14. Sobald die Elektrodenabdeckungen 5 an ihren den Elektroden abgewandten Seiten zusammengelegt werden, stehen die Elektroden 1 über die kleine Ausnehmung 15 zueinander in Verbindung. Wichtig dabei ist, dass beim Zusammenlegen der Elektrodenabdeckungen 5 darauf geachtet wird, dass die Ausnehmungen 14, 15 fluchten, beziehungsweise die kleine Ausnehmung 15 sich tatsächlich in der großen Ausnehmung 14 befindet. Dies kann beispielsweise über Positionierungshilfen 18 geschehen, welche in den Elektrodenabdeckungen 5 angeordnet sind. So kann zum Beispiel eine Vorrichtung die Elektrodenabdeckungen 5 an den Positionierungshilfen 18 aufnehmen und diese in einer korrekten Lage miteinander verbinden. Die Positionierungshilfe 18 kann auch durch die Außenform der Elektrodenabdeckung 5 ausgestaltet und muss nicht als Ausnehmung ausgebildet sein.

## Patentansprüche

1. Elektrodenset (30), insbesondere für einen Defibrillator (35), beinhaltend zumindest zwei Elektroden (1 a, 1 b), jeweils bestehend aus
- einer Trägerschicht (2),
- einer leitfähigen Kontaktschicht (3),
- einer leitfähigen Gelschicht (4),
- einer nichtleitenden Elektrodenabdeckung (5),
wobei im Lagerzustand des Elektrodensets (30) die Elektrodenabdeckungen (5), auf der von der Gelschicht (4) der zumindest zwei Elektroden (1 a, 1 b) abgewandten Seite, zumindest abschnittsweise flächig aneinander liegen, **dadurch gekennzeichnet, dass** sich die Gelschichten (4) der Elektroden (1a, 1 b) abschnittsweise über zumindest zwei unterschiedlich große Ausnehmungen (14,15) in den Elektrodenabdeckungen (5) direkt berühren.

2. Elektrodenset nach Anspruch 1, **dadurch gekennzeichnet, dass** die Anzahl auf zwei Elektroden (1a, 1b) pro Elektrodenset (30) beschränkt ist.

3. Elektrodenset nach Anspruch 1 oder 2, **dadurch gekennzeichnet, dass** im Lagerzustand des Elektrodensets (30) ein Stromleiter (6) an den Elektroden (1a, 1 b) befestigt ist.

4. Elektrodenset nach einem der Ansprüche 1 bis 3, **dadurch gekennzeichnet, dass** im Lagerzustand des Elektrodensets (30) eine gasdichte Verpackung (10) das Elektrodenset (30) umhüllt.

5. Elektrodenset nach einem der Ansprüche 3 bis 4, **dadurch gekennzeichnet, dass** im Lagerzustand des Elektrodensets (30) der Stromleiter (6) aus der gasdichten Verpackung (10) herausgeführt ist.

6. Elektrodenset nach einem der Ansprüche 1 bis 5, **dadurch gekennzeichnet, dass** für jede Elektrode (1 a, 1 b) je eine gesonderte Elektrodenabdeckung (5) vorgesehen ist. (Fig. 8)

7. Elektrodenset nach einem der Ansprüche 1 bis 5, **dadurch gekennzeichnet, dass** die Elektrodenabdeckungen (5) eine gemeinsame - vorzugsweise einstückige - Elektrodenabdeckung (5) bilden. (Fig. 4)

8. Elektrodenset nach Anspruch 7, **dadurch gekennzeichnet, dass** die zumindest abschnittsweise im Wesentlichen ebenen Elektrodenabdeckungen (5) miteinander über eine Faltlinie (13) ständig in Verbindung stehen. (Fig. 4)

9. Elektrodenset nach einem der Ansprüche 1 bis 8, **dadurch gekennzeichnet, dass** die Gesamtoberfläche der Elektrodenabdeckungen (5) zumindest das Zweifache der Fläche der Gelschichten (4) der Elektroden (1a, 1b) beträgt.

10. Elektrodenset nach einem der Ansprüche 1 bis 9, **dadurch gekennzeichnet, dass** jene Flächen der Elektrodenabdeckungen (5) - gegenüberliegend der Flächen, auf welchen die Elektroden (1 a, 1 b) befestigt sind - frei von Elektroden (1a, 1 b) sind.

11. Elektrodenset nach einem der Ansprüche 1 bis 10, **dadurch gekennzeichnet, dass** die Elektrodenabdeckungen (5) zumindest je eine Ausnehmung (14,15) in einem Aufnahmebereich (20a, 20b) aufweisen.

12. Elektrodenset nach Anspruch 11, **dadurch gekennzeichnet, dass** die Elektrodenabdeckungen (5) zumindest eine große Ausnehmung (14) in einem Aufnahmebereich (20a, 20b) und zumindest eine kleine Ausnehmung (15) in einem weiteren Aufnahmebereich (20a, 20b) aufweist.

13. Elektrodenset nach Anspruch 12, **dadurch gekennzeichnet, dass** im Lagerzustand des Elektrodensets (30) die zumindest eine kleine Ausnehmung (15) mit der zumindest einen großen Ausnehmung (14) fluchtet.

14. Verfahren zur Herstellung eines Elektrodensets (30), insbesondere für einen Defibrillator (35), nach einem der Ansprüche 1 bis 13, **dadurch gekennzeichnet, dass** folgende Verfahrensschritte beinhaltet sind:
- Fertigung der unterschiedlichen Ausnehmungen (14, 15) in dem auf einer Bahn laufenden Material der Elektrodenabdeckung (5) durch zumindest eine Lochstanze (21) oder durch einen Laser.
- Auftragen des Grundmaterials der unausgestanzten Elektroden (1a, 1b) oder Auftragen der fertigen Elektroden (1a, 1 b) auf das auf einer Bahn laufende Material der Elektrodenabdeckung (5).
- Fertigen der Form der Elektroden (1a, 1b) durch zumindest eine Elektrodenstanze (22) oder einen Laser mit Abziehen des Restmaterials und Ausschneiden der Form der Elektrodenabdeckungen (5) mit den vorzugsweise paarweise aufgeklebten Elektroden (1a, 1b) und zeitgleichem Perforieren der Faltlinie (13) durch die Kartenstanze (23) oder einen Laser, wobei das Ausschneiden der Form der Elektrodenabdeckung (5) mit den paarweise aufgeklebten Elektroden (1a, 1 b) und zeitgleichem Perforieren der Faltlinie (13) durch die Kartenstanze auch in einem separaten Arbeitsschritt (23) erfolgen oder im Falle getrennter Elektrodenabdeckungen (5) entfallen kann.
- Abziehen des Restmaterials der Elektrodenabdeckung (5) und der gefertigten Ausnehmungen (14,15) in einem separaten Arbeitsschritt oder während einem der anderen Arbeitsschritte.

15. Verfahren zur Prüfung eines Leitwertes (S) einer Gelschicht (4) in einem Elektrodenset (30) nach einem der Ansprüche 1 bis 13, **dadurch gekennzeichnet, dass** ein Kontrollstrom (K) ausgehend von einer elektrischen Vorrichtung, vorzugsweise einem Defibrillator (35), durch das im Lagerzustand befindliche, verpackte Elektrodenset (30) geleitet wird, wobei sich die zumindest eine kleine Ausnehmung (15) vollständig im Bereich der zumindest einen großen Ausnehmung (14) befindet und somit eine durch die Fläche der kleinen Ausnehmung (15) definierte Kontaktfläche (16) der Elektroden (1 a, 1 b) zueinander bildet, durch welche der Kontrollstrom (K) die Elektrodenabdeckung (5) passieren kann und der Leitwert (S) ermittelbar wird.

## Claims

1. An electrode set (30), in particular for a defibrillator (35), including at least two electrodes (1a, 1b) each comprising
- a carrier layer (2),
- a conductive contact layer (3),
- a conductive gel layer (4), and
- a non-conductive electrode cover (5),
wherein in the storage state of the electrode set (30) the electrode covers (5) lie against each other in planar relationship at least portion-wise, on the side that is remote from the gel layer (4) of the at least two electrodes (1 a, 1 b), **characterised in that** the gel layers (4) of the electrodes (1 a, 1 b) are directly in contact with each other in portion-wise manner by way of at least two differently sized openings (14, 15) in the electrode covers (5).

2. An electrode set as set forth in claim 1 **characterised in that** the number is limited to two electrodes (1 a, 1 b) per electrode set (30).

3. An electrode set as set forth in claim 1 or claim 2 **characterised in that** in the storage state of the electrode set (30) a current conductor (6) is fixed to the electrodes (1a,1b).

4. An electrode set as set forth in one of claims 1 through 3 **characterised in that** in the storage state of the electrode set (30) a gas-tight packaging (10) encloses the electrode set (30).

5. An electrode set as set forth in one of claims 3 and 4 **characterised in that** in the storage state of the electrode set (30) the current conductor (6) is passed out of the gas-tight packaging (10).

6. An electrode set as set forth in one of claims 1 through 5 **characterised in that** a respective separate electrode cover (5) is provided for each electrode (1 a, 1 b). (Figure 8)

7. An electrode set as set forth in one of claims 1 through 5 **characterised in that** the electrode covers (5) form a common - preferably one-piece - electrode cover (5). (Figure 4)

8. An electrode set as set forth in claim 7 **characterised in that** the electrode covers (5) which are at least portion-wise substantially flat are constantly connected to each other by way of a fold line (13). (Figure 4)

9. An electrode set as set forth in one of claims 1 through 8 **characterised in that** the overall surface area of the electrode covers (5) is at least double the area of the gel layers (4) of the electrodes (1a, 1b).

10. An electrode set as set forth in one of claims 1 through 9 **characterised in that** those surfaces of the electrode covers (5) - in opposite relationship to the surfaces on which the electrodes (1 a, 1 b) are fixed - are free of electrodes (1 a, 1 b).

11. An electrode set as set forth in one of claims 1 through 10 **characterised in that** the electrode covers (5) have at least one respective opening (14, 15) in a receiving region (20a, 20b).

12. An electrode set as set forth in claim 11 **characterised in that** the electrode covers (5) have at least one large opening (14) in a receiving region (20a, 20b) and at least one small opening (15) in a further receiving region (20a, 20b).

13. An electrode set as set forth in claim 12 **characterised in that** in the storage state of the electrode set (30) the at least one small opening (15) aligns with the at least one large opening (14).

14. A process for producing an electrode set (30), in particular for a defibrillator (35), as set forth in one of claims 1 through 13, **characterised in that** it comprises the following process steps:
- production of the different openings (14, 15) in the material extending on a web of the electrode cover (5) by at least one hole stamping punch (21) or by a laser,
- applying the basic material of the electrodes (1a, 1b) which are not punched out or applying the finished electrodes (1 a, 1b) to the material of the electrode cover (5), that runs on a web,
- finishing the shape of the electrodes (1 a, 1b) by at least one electrode punch (22) or a laser with removal of the residual material and cutting out of the shape of the electrode covers (5) with the electrodes (1 a, 1 b) which are preferably glued on in pair-wise relationship and simultaneously perforating the fold line (13) by the card punch (23) or a laser, wherein the operation of cutting out the shape of the electrode cover (5) with the electrodes (1a, 1b) which are glued on in pair-wise relationship and simultaneous perforation of the fold line (13) by the card punch are also effected in a separate working step (23) or in the case of separate electrode covers (5) can be omitted, and
- pulling off the residual material of the electrode cover (5) and the produced openings (14, 15) in a separate working step or during one of the other working steps.

15. A process for checking a conductance (S) of a gel layer (4) in an electrode set (30) as set forth in one of claims 1 through 13, **characterised in that** a checking current (K) is passed from an electrical device, preferably a defibrillator (35), through the packaged electrode set (30) disposed in the storage state, wherein the at least one small opening (15) is completely in the region of the at least one large opening (14) and thus forms a contact surface (16) of the electrodes (1a, 1b) relative to each other, which contact surface is defined by the area of the small opening (15) and through which the checking current (K) can pass the electrode cover (5) and the conductance (S) can be ascertained.

## Revendications

1. Jeu d'électrodes (30), en particulier pour un défibrillateur (35), contenant au moins deux électrodes (1a, 1 b), constituées respectivement
- d'une couche de support (2),
- d'une couche de contact (3) conductrice,
- d'une couche de gel (4) conductrice,
- d'un recouvrement d'électrode (5) non conducteur,
dans lequel dans l'état d'entreposage du jeu d'électrodes (30), les recouvrements d'électrode (5), reposent au moins par endroits à plat les uns au niveau des autres sur le côté opposé à la couche de gel (4) des deux électrodes (1a, 1b) ou plus,
**caractérisé en ce que** les couches de gel (4) des électrodes (1a, 1b) sont en contact direct par endroits par l'intermédiaire au moins deux évidements (14, 15) de taille différente dans les recouvrements d'électrode (5).

2. Jeu d'électrodes selon la revendication 1, **caractérisé en ce que** le nombre de deux électrodes (1a, 1 b) est limité par jeu d'électrodes (30).

3. Jeu d'électrodes selon la revendication 1 ou 2, **caractérisé en ce que** dans l'état d'entreposage du jeu d'électrodes (30), un conducteur de courant (6) est fixé au niveau des électrodes (1a, 1b).

4. Jeu d'électrodes selon l'une quelconque des revendications 1 à 3, **caractérisé en ce que** dans l'état d'entreposage du jeu d'électrodes (30), un emballage (10) étanche aux gaz enveloppe le jeu d'électrodes (30).

5. Jeu d'électrodes selon l'une quelconque des revendications 3 à 4, **caractérisé en ce que** dans l'état d'entreposage du jeu d'électrodes (30), le conducteur de courant (6) est guidé en dehors de l'emballage (10) étanche aux gaz.

6. Jeu d'électrodes selon l'une quelconque des revendications 1 à 5, **caractérisé en ce que** pour chaque électrode (1a, 1 b), respectivement un recouvrement d'électrode (5) séparé est prévu (figure 8).

7. Jeu d'électrodes selon l'une quelconque des revendications 1 à 5, **caractérisé en ce que** les recouvrements d'électrode (5) forment un recouvrement d'électrode (5) commun - de préférence d'un seul tenant - (figure 4).

8. Jeu d'électrodes selon la revendication 7, **caractérisé en ce que** les recouvrements d'électrode (5) au moins par endroits sensiblement plans sont reliés les uns aux autres en permanence par l'intermédiaire d'une ligne de pliage (13) (figure 4).

9. Jeu d'électrodes selon l'une quelconque des revendications 1 à 8, **caractérisé en ce que** la surface totale des recouvrements d'électrode (5) représente au moins le double de la surface des couches de gel (4) des électrodes (1a, 1 b).

10. Jeu d'électrodes selon l'une quelconque des revendications 1 à 9, **caractérisé en ce que** ces surfaces précisément des recouvrements d'électrode (5) - faisant face aux surfaces, sur lesquelles les électrodes (1a, 1 b) sont fixées - sont sans électrodes (1a, 1 b).

11. Jeu d'électrodes selon l'une quelconque des revendications 1 à 10, **caractérisé en ce que** les recouvrements d'électrode (5) présentent au moins respectivement un évidement (14, 15) dans une zone de réception (20a, 20b).

12. Jeu d'électrodes selon la revendication 11, **caractérisé en ce que** les recouvrements d'électrode (5) présentent au moins un évidement (14) de grande taille dans une zone de réception (20a, 20b) et au moins un évidement (15) de petite taille dans une autre zone de réception (20a, 20b).

13. Jeu d'électrodes selon la revendication 12, **caractérisé en ce que** dans l'état d'entreposage du jeu d'électrodes (30), l'au moins un évidement (15) de petite taille est aligné avec l'au moins un évidement (14) de grande taille.

14. Procédé servant à fabriquer un jeu d'électrodes (30), en particulier pour un défibrillateur (35), selon l'une quelconque des revendications 1 à 13, **caractérisé en ce que** des étapes de procédé qui suivent sont contenues :
- la confection des différents évidements (14, 15) dans le matériau circulant sur une voie du recouvrement d'électrode (5) par au moins une machine à poinçonner (21) ou par un laser ;
- l'application du matériau de base des électrodes (1a, 1b) non découpées ou l'application des électrodes (1a, 1 b) fin prêtes sur le matériau, circulant sur une voie, du recouvrement d'électrode (5) ;
- la confection de la forme des électrodes (1a, 1b) par au moins une poinçonneuse d'électrode (22) ou par un laser, avec retrait du matériau restant et découpage de la forme des recouvrements d'électrode (5) avec les électrodes (1 a, 1b) collées de préférence par paire et, dans le même temps, avec perforation de la ligne de pliage (13) par la poinçonneuse de carte (23) ou par un laser, dans lequel le découpage de la forme du recouvrement d'électrode (5) avec les électrodes (1a, 1b) collées par paire et avec la perforation dans le même temps de la ligne de pliage (13) par la poinçonneuse de carte est effectué également lors d'une étape de travail (23) séparée ou peut être supprimé dans le cas de recouvrements d'électrode (5) séparés ;
- le retrait du matériau restant du recouvrement d'électrode (5) et des évidements (14, 15) confectionnés lors d'une étape de travail séparée ou au cours d'une des autres étapes de travail.

15. Procédé servant à vérifier une conductance (S) d'une couche de gel (4) dans un jeu d'électrodes (30) selon l'une quelconque des revendications 1 à 13, **caractérisé en ce qu'**un courant de contrôle (K) est acheminé en partant d'un dispositif électrique, de préférence d'un défibrillateur (35), à travers le jeu d'électrodes (30) emballé se trouvant dans l'état d'entreposage, dans lequel l'au moins un évidement (15) de petite taille se trouve totalement dans la zone de l'au moins un évidement (14) de grande taille et forme ainsi une surface de contact (16), définie par la surface de l'évidement (15) de petite taille, des électrodes (1a, 1b), par laquelle le courant de contrôle (K) peut traverser le recouvrement d'électrode (5) et que la conductance (S) peut être déterminée.
